(19) Europäisches Patentamt
European Patent Office
Office européen des brevets

(11) Veröffentlichungsnummer: **0 116 515**
**B1**

(12) # EUROPÄISCHE PATENTSCHRIFT

(45) Veröffentlichungstag der Patentschrift: **21.09.88**

(51) Int. Cl.[4]: **A 01 N 43/82,** C 07 D 401/04 // (C07D401/04, 285:00, 213:00)

(21) Anmeldenummer: **84810024.4**

(22) Anmeldetag: **13.01.84**

(54) **Schädlingsbekämpfungsmittel.**

(30) Priorität: **19.01.83 CH 300/83**

(43) Veröffentlichungstag der Anmeldung: **22.08.84 Patentblatt 84/34**

(45) Bekanntmachung des Hinweises auf die Patenterteilung: **21.09.88 Patentblatt 88/38**

(84) Benannte Vertragsstaaten: **AT BE CH DE FR GB IT LI NL SE**

(56) Entgegenhaltungen:
**CH-A-411 906**
**DD-C-157 664**
**US-A-2 733 245**
**US-A-2 744 908**

**CHEMICAL ABSTRACTS, Band 72, Nr. 17, 27. April 1970, Seite 388, Nr. 90374p, Columbus, Ohio, USA N.IKEDA et al.: Syntheses of 1,3,4-thiadiazole derivatives".**
**JOURNAL OF ORGANIC CHEMISTRY, Band 45, Nr. 19, 12. September 1980, Seiten 3750-3753, American Chemical Society, Easton, PA, USA YANG-I LIN et al.: "New synthesis of 1.3,4-thiadiazoles".**
**JOURNAL OF THE AMERICAN CHEMICAL SOCIETY, Band 80, 5. Oktober 1958, Seiten 5201-5203, Easton, PA, USA, C.AINSWORTH: " The investigation of some substituted 1,3,4-thiadiazoles".**

(73) Patentinhaber: **CIBA- GEIGY AG, Klybeckstrasse 141, CH- 4002 Basel (CH)**

(72) Erfinder: **Kristiansen, Odd, Dr., Delligrabenstrasse 7, CH- 4313 Möhlin (CH)**
Erfinder: **Drabek, Jozef, Dr., Benkenstrasse 12, CH- 4104 Oberwil (CH)**

EP 0 116 515 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft die Verwendung von 2-(3-Pyridyl)-1,3,4-thiadiazolen und 5-(3-Pyridyl)-1,2,4-thiadiazolen in der Schädlingsbekämpfung sowie neue 2-(3-Pyridyl)-1,3,4-thiadiazole und 5-(3-Pyridyl)-1,2,4-thiadiazole und Verfahren zu ihrer Herstellung.

Ein Gegenstand der vorliegenden Erfindung ist die Verwendung von 2-(3-Pyridyl)-1,3,4-thiadiazolen und 5-(3-Pyridyl)-1,2,4-thiadiazolen der Formel I

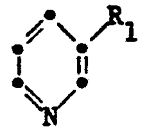 (I) ,

worin $R_1$ 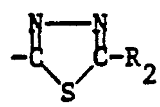 oder 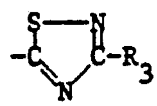

und $R_2$ und $R_3$ Wasserstoff oder $C_1$-$C_4$-Alkyl bedeuten, oder eines diese Verbindung enthaltenden Mittels zur Bekämpfung von Pflanzen und Tiere befallenden Insekten und Vertretern der Ordnung Akarina.

Bevorzugt wird die Verwendung solcher Verbindungen der Formel I, worin $R_2$ und $R_3$ Wasserstoff oder $C_1$-$C_3$-Alkyl bedeuten.

Ein weiterer Gegenstand der Erfindung sind neuartige unter die vorstehende Formel I fallende 2-(3-Pyridyl)-1,3,4-thiadiazole und 5-(3-Pyridyl)-1,2,4-thiadiazole, worin $R_2$ Wasserstoff oder $C_2$-$C_4$-Alkyl und $R_3$ $C_1$-$C_4$-Alkyl bedeuten.

Die Alkylgruppen bei $R_2$ und $R_3$ können geradkettig oder verzweigt sein; diese Gruppen sind: Methyl, Äthyl, n-Propyl, Isopropyl, n-, i-, sek.- oder tert.-Butyl.

Hervorzuheben sind neuartige unter Formel I fallende Verbindungen, die dadurch gekennzeichnet sind, dass $R_1$ die oben angegebene Bedeutung hat,
$R_2$ Wasserstoff oder $C_2$-$C_3$-Alkyl und
$R_3$ $C_1$-$C_3$-Alkyl bedeuten.

Wegen ihrer pestiziden Wirkung besonders bevorzugt sind neuartige Verbindungen der Formel I, worin

$R_1$ 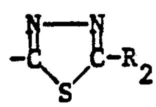

bedeutet und $R_2$ Wasserstoff oder $C_2$-$C_3$-Alkyl bedeutet.

Die Verbindungen der Formel I können nach an sich bekannten Verfahren [vgl. u.a. J.Am.Chem.Soc.80, 5201 (1958); J.Het.Chem. 1319 (1981); J.Org.Chem. 45, No. 19, 3750 ff. (1980)], z. B. wie folgt hergestellt werden:

1) 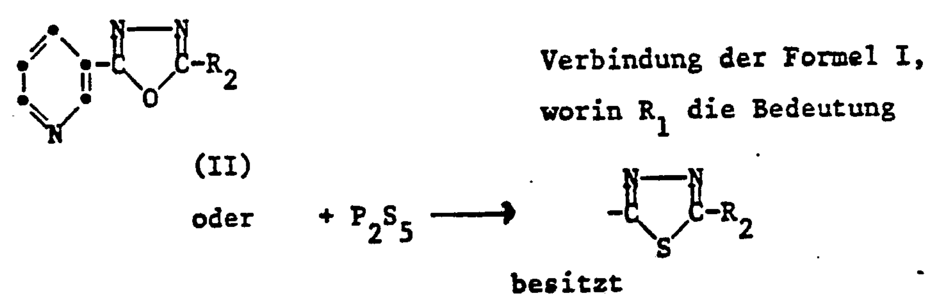


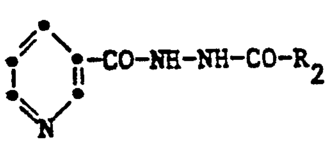 III

2) 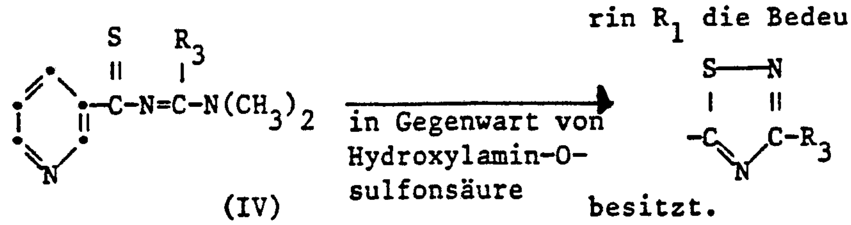


(IV)

Verbindung der Formel I, worin $R_1$ die Bedeutung ... besitzt.

In den Formeln II bis IV haben $R_2$ und $R_3$ die unter Formel I angegebenen Bedeutungen.

Die Verfahren 1 und 2 werden bei normalem Druck, bei einer Temperatur von 5° bis 180° C, vorzugsweise von 20° bis 160° C und gegebenenfalls in einem Lösungs- oder Verdünnungsmittel durchgeführt.

Als Lösungs- oder Verdünnungsmittel eignen sich z. B. Äther und ätherartige Verbindungen wie Diäthyläther, Di-isopropyläther, Dioxan, Tetrahydrofuran; aliphatische und aromatische Kohlenwasserstoffe, insbesondere Benzol, Toluol, Xylole; Ketone wie Aceton, Methyläthylketon und Cyclohexanon.

Die Ausgangsstoffe der Formeln II, III und IV sind zumeist bekannt, bzw. können, falls sie neu sind, analog bekannten Methoden hergestellt werden.

So kann man z. B. die 2-Pyridyl-oxdiazole der Formel II durch Umsetzung von Nikotinsäurehydrazid mit Orthoestern erhalten [vgl. J.Am.Chem.Soc.77, 1148 (1955); Die 2-Acyl-Nikotinsäurehydrazide der Formel III sind durch Umsetzung von Nikotinsäurehydrazid mit entsprechenden reaktiven Carbonsäurederivaten zugänglich [vgl.J.Am. Chem.Soc.75, 1933 (1953)].

Zu den N'-(3-Pyridyl)-thiocarbonyl-N,N-dimethylamidinen der Formel IV kann man durch Umsetzung von Thionikotinsäureamid der Formel V mit entsprechenden Amidacetalen der Formel VI worin $R_3$ die oben angegebene Bedeutung hat, gelangen [vgl.J.Org.Chem. 45, No. 19, 3751 (1980)]:

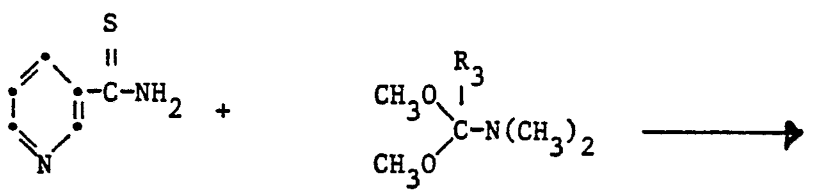

(V) (VI) (IV).

Die Herstellung von 2-Phenyl-5-(3-pyridyl)-1, 3, 4-thiadiazolen und deren Verwendung als UV-Schutzmittel, optische Aufheller und Farbstoffzwischenprodukte wird in der schweizerischen Patentschrift CH-A-411 906 beschrieben. In der US-A-2 733 245 werden unter anderem substituierte 5-Pyridyl-1,3,4-oxadiazole mit pharmakologischer Wirkung offenbart. Auf die larvizide Wirksamkeit von 2-Phenyl-5-alkylmercapto-1,3,4-thiadiazolen wird in der US-A-2 744 908 hingewiesen.

Es ist weiterhin aus der veröffentlichten europäischen Patentanmeldung EP-A-0 062 612 und der US-A-4 260 765 bekannt, dass substituierte 3-Pyridyl-thiazoline bzw. -thiazole insektizide Wirkung, insbesondere gegen saugende Insekten, wie Blattläuse, aufweisen. Ferner ist das 5-(3-Pyridyl)-1,2,4-thiadiazol und dessen Herstellung aus J.Org.Chem. 1980, Vol. 45, S. 3751, und das 2-Methyl-5-(3-pyridyl)-1,3,4-thiadiazol aus C.A. Vol. 72, 90 374 p. (1970) bekannt; dort werden jedoch keine Angaben über eine mögliche biologische Wirksamkeit dieser Verbindungen gemacht. Überraschenderweise wurde nunmehr gefunden, dass die erfindungsgemäss vorgeschlagene Klasse der 3-Pyridylthiadiazol-Verbindungen, die bis auf die vorerwähnten 5-(3-Pyridyl)-thiadiazole neuartig sind, gute pestizide, vor allem aphizide, Wirksamkeit aufweist.

Die Verbindungen der Formel I eignen sich zur Bekämpfung von Schädlingen an Tieren, Pflanzen und im Boden, wie z. B. zur Bekämpfung von Insekten der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von phytopathogenen Milben und Zecken der Ordnung Acarina.

Vor allem eignen sich Verbindungen der Formel I zur Bekämpfung von pflanzenschädigenden Insekten, insbesondere pflanzenschädigenden Frassinsekten, in Zier- und Nutzpflanzungen, insbesondere in Baumwollkulturen (z. B. Spodoptera littoralis und Heliothis virescens) und Gemüsekulturen (z. B. Leptinotarsa decemlineata und Myzus persicae).

In diesem Zusammenhang ist hervorzuheben, dass die genannten Verbindungen sich sowohl durch eine stark ausgeprägte systematische als auch Kontakt- und Blattpenetrationswirkung gegen saugende Insekten und vor allem gegen Insekten der Familie Aphididae (wie z. B. Aphis fabae, Aphis craccivora und Myzus persicae), welche sich mit bekannten Mitteln nur schwierig bekämpfen lassen, auszeichnen.

Wirkstoffe der Formel I zeigen auch eine sehr günstige Wirkung gegen Fliegen, wie z. B. Musca domestica und Mückenlarven. Ferner zeichnen sie sich durch eine breite ovizide und ovolarvizide Wirkung aus und besitzen eine wertvolle Wirkung gegen ektoparasitäre Milben und Zecken z. B. der Familien Ixodidae,

Argasidae und Dermanyssidae.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wir Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen cignen sich Alkali-, Erdalkali- oder gegebenenfalls substituierte Ammoniumsalze von höheren Fettsäuren ($C_{10}$-$C_{22}$), wie z. B. die Na- oder K-Salze der Öl- oder Stearinsäure, oder von natürlichen Fettsäuregemischen, die z. B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8 - 22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor z. B. das

Stearyltrimethylammoniumchlorid oder das Benzyldi-(2-chloräthyl)-äthylamnoniumbromid.

Die in der Formulierungstechnik gebräuchlichen Tenside sind u.a. in folgenden Publikationen beschrieben:

"Mc Cutcheon's Detergents und Emulsifiers Annual" MC Publishing Corp., Ringwood, New Jersey, 1979; Dr. Helmut Stacke: "Tensid Taschenbuch", Carl Hauser Verlag, München/ Wien, 1984.

Die erfindungsgemäss verwendeten pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides. Während als Handelsware eher konzentrierte Mittel bevorzugt werden, verwendet der Endverbraucher in der Regel verdünnte Mittel.

Die erfindungsgemäss zu verwendenden Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

<u>Formulierungsbeispiele für flüssige Wirkstoffe der Formel I</u>
<u>(% = Gewichtsprozent)</u>

| 1. <u>Emulsions-Konzentrate</u> | a) | b) | c) |
|---|---|---|---|
| Wirkstoff | 25 % | 40 % | 50 % |
| Ca-Dodecylbenzolsulfonat | 5 % | 8 % | 6 % |
| Ricinusöl-polyäthylenglykoläther (36 Mol ÄO) | 5 % | - | - |
| Tributylphenyl-polyäthylenglykoläther (30 Mol ÄO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. <u>Lösungen</u> | a) | b) | c) | d) |
|---|---|---|---|---|
| Wirkstoff | 80 % | 10 % | 5 % | 95 % |
| Äthylenglykol-monomethyläther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160 - 190° C) | - | - | 94 % | - |

Die Lösungen sind zur Anwendung in Form kleinster Tropfen geeignet.

| 3. <u>Granulate</u> | a) | b) |
|---|---|---|
| Wirkstoff | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 4. <u>Stäubemittel</u> | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I
(% = Gewichtsprozent)

| 5. Spritzpulver | a) | b) | c) |
|---|---|---|---|
| Wirkstoff | 25 % | 50 % | 75 % |
| Na-Ligninsulfonat | 5 % | 5 % | - |
| Na-Laurylsulfat | 3 % | - | 5 % |
| Na-Diisobutylniphthalinsulfonat | - | 6 % | 10 % |
| Octylphenolpolyäthylenglykol äther (7 - 8 Mol ÄO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
|---|---|
| Wirkstoff | 10 % |
| Octylphenolpolyäthylenglykoläther (4 - 5 Mol ÄO) | 3 % |
| Ca-Dodecylbenzolsulfonat | 3 % |
| Ricinusölpolyglykoläther (36 Mol ÄO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| 8. Extruder-Granulat | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 9. Umhüllungs-Granulat | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 10. Suspensions-Konzentrat | |
|---|---|
| Wirkstoff | 40 % |
| Äthylenglykol | 10 % |
| Nonylphenolpolyäthylenglykol äther (15 Mol ÄO) | 6 % |
| Na- Ligninsulfonat | 10 % |
| Carboxymethylcellulose | 1 % |
| 37-%-ige wässrige Formaldehyd-Lösung | 0,2 % |
| Silikonöl in Form einer 75-%-igen wässrigen Emulsion | 0,8 % |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration

hergestellt werden können.

**Beispiel 1:**

Zur Herstellung des Ausgangsproduktes wird ein Gemisch aus 15,6 g Nikotinsäurehydrazid, 70 g Orthoameisensäuretriäthylester und 1 Spatelspitze Kaliumhydrogensulfat während 2 Stunden bei einer Badtemperatur von 155°C gerührt. Das gebildete Äthanol wird fortlaufend während der Reaktion abgezogen. Anschliessend wird der überschüssige Orthoester am Wasserstrahlvakuum abdestilliert. Das erhaltene Ausgangsprodukt 2-(3-Pyridyl)-1,3,4-oxadiazol hat einen Schmelzpunkt von 80 - 82°C.

<u>2-(3-Pyridyl)-1,3,4-thiadiazol</u>

Ein Gemisch aus 5,5 g des wie oben hergestellten 2-(3-Pyridyl)-1,3,4-oxadiazols, 8,5 g Phosphorpentasulfid und 70 ml Xylol wird 16 Stunden bei 150° bis 160°C gerührt. Nach dem Abkühlen wird mit 50 ml Wasser versetzt und nochmals 5 Minuten gerührt. Das Reaktionsgemisch wird filtriert. Der Rückstand wird in 100 ml 10-%-iger Natronlauge aufgenommen und mit Chloroform extrahiert. Die organische Phase wird mit Wasser gewaschen, getrocknet und eingedampft.

Nach säulenchromatographischer Reinigung [Kieselgel; Eluiermittel Chloroform/Äthanol (95 : 5)] erhält man das 2-(3-Pyridyl)-1,3,4-thiadiazol der Formel

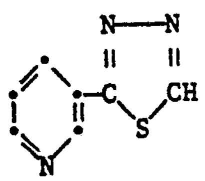

(Verbindung Nr. 1)

als vorgereinigtes Rohprodukt mit einem Schmelzpunkt von 64 - 67°C.

**Beispiel 2:**

Zu einer Lösung von 22 g N'-Formylnikotinsäurehydrazid in 150 ml Pyridin werden 28 g Phosphorpentasulfid in Portionen zugefügt, wobei die Temperatur auf 65°C ansteigt. Nach 5 Minuten wird das Gemisch auf 110°C erhitzt und für 2 Stunden bei dieser Temperatur gerührt. Anschliessend wird das Pyridin am Wasserstrahlvakuum abdestilliert, der Rückstand in Chloroform aufgenommen, mit 5-%-iger Natronlauge und mit Wasser ausgeschüttelt. Nach dem Trocknen der organischen Phase und Eindampfen des Lösungsmittels wird das reine 2-(3-Pyridyl)-1,3,4-thiadiazol als hellgelbes Pulver mit einem Schmelzpunkt von 78 - 79°C erhalten.

**Beispiel 3:**

Auf analoge Weise wird, wie in den vorhergehenden Beispielen 1 und 2 beschrieben auch die Verbindung Nr. 2 der Formel

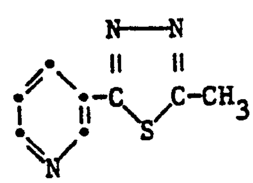

mit einem Schmelzpunkt von 124 - 126°C sowie die Verbindung Nr. 3 der Formel

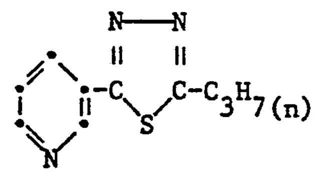

mit einem Schmelzpunkt von 61 - 63° C hergestellt.

**Beispiel 4:**

Zu einer Lösung von 10,4 g N'-(3-Pyridyl)-thiocarbonyl-N,N-dimethyl-acetamidin in 200 ml Äthanol und 120 ml Methanol werden bei 20° C 6,22 g Hydroxylamin-D-sulfonsäure zugetropft. Nach einstündigem Rühren wird das Reaktionsgemisch eingeengt und der Rückstand in Methylenchlorid aufgenommen. Die Lösung wird je mit 250 ml Wasser und 250 ml In Natronlauge ausgeschüttelt und über $Na_2SO_4$ getrocknet. Nach dem Abdestillieren des Methylenchlorides wird der Rückstand bei 100° C/0,035 Pa destilliert.

Man erhält das 3-Methyl-5-(3-pyridyl)-1,2,4-thiadiazol der Formel

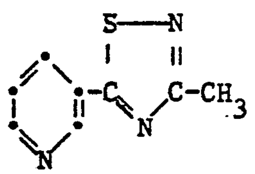

(Verbindung Nr. 4)

mit einem Schmelzpunkt von 65° C.

**Beispiel 5:**

Auf analoge Weise wie im vorhergehenden Beispiel 4 angegeben wird, ausgehend vom N'-(3-Pyridyl)-thiocarbonyl-N,N-dimethylformamidin auch die Verbindung Nr. 5 der Formel

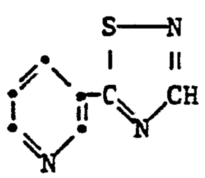

- Schmelzpunkt 81 - 83° C - hergestellt.

**Beispiel 6:**

Insektizide systemische Wirkung in Erde: Myzus persicae

Bewurzelte Kohlpflanzen werden in Töpfe, welche 60 ccm Erde enthalten, verpflanzt. Anschliessend werden 50 ml einer Versuchslösung enthaltend 0,75 ppm bzw. 3 ppm der zu prüfenden Verbindung, direkt auf die Erde aufgegossen.

Nach 24 Stunden werden auf die oberirdischen Pflanzenteile Blattläuse (Myzus persicae) gesetzt, und über die Pflanzen wird ein unten zugeschnürter Plastikzylinder gestülpt, um die Läuse vor einer eventuellen Kontakt- oder Gaswirkung der Testsubstanz zu schützen. Die Auswertung der erzielten Abtötung erfolgt 48 Stunden und dann 7 Tage nach Versuchsbeginn. Pro Konzentrationsdosis der Testsubstanz werden zwei Pflanzen, je eine in einem separaten Topf, verwendet. Der Versuch wird bei 25° C und 70 % relativer Luftfeuchtigkeit durchgeführt.

Die Verbindung Nr. 1 gemäss Beispiel 1 bzw. 2 zeigt bei 0,75 ppm 80 - 100-%-ige Wirkung (Mortalität) gegen Myzus. Bei einer Dosierung von 3 ppm ist die Verbindung noch nach 28 Tagen zu 80 - 100 % wirksam.

**Beispiel 7:**

Insektizide systemische Wirkung in Wasser: Aphis craccivora

Erbsenkeimlinge, die 24 Stunden vor Beginn des Versuches mit den Blattläusen infestiert worden sind, werden in 20 ml einer wässrigen Brühe gestellt, die 12,5 ppm des zu prüfenden Wirkstoffes enthält. Die wässrige Brühe wird aus einem Emulsionskonzentrat oder einer benetzbaren Pulverzubereitung des Wirkstoffes hergestellt und befindet sich in einem Gefäss, das mit einem Löcher aufweisenden Plasticdeckel abgeschlossen ist. Die Wurzel der infestierten Erbsenpflanze wird jeweils durch ein Loch in dem Plastikdeckel in die Brühe geschoben. Das Loch wird dann mit Watte abgedichtet, um die Pflanze zu fixieren und den Einfluss der Gasphase aus der Brühe auszuschalten.

Der Versuch wird bei 20°C und 60 % relativer Luftfeuchtigkeit durchgeführt. Nach zwei Tagen wird auf die Anzahl der nicht mehr saugfähigen Testtiere im Vergleich zu unbehandelten Kontrollen bonitiert. Damit wird festgestellt, ob der über die Wurzel aufgenommene Wirkstoff die Blattläuse an den oberen Pflanzenteilen abtötet.

Verbindung Nr. 4 gemäss Beispiel 4 zeigt im obigen Versuch eine 80 - 100-%-ige systemische Wirkung (Abtötung) gegen Aphis craccivora bei 12,5 ppm.

**Beispiel 8:**

Insektizide Kontaktwirkung: Myzus persicae

Etwa 4 cm hohe, in Wasser angezogene Erbsenkeimlinge werden vor Versuchsbeginn je mit ca. 200 Individuen der Spezies Myzus persicae besiedelt. Die so behandelten Pflanzen werden 24 Stunden später mit einer wässrigen Suspension enthaltend 100 ppm der zu prüfenden Verbindung bis zur Tropfnässe besprüht. Man verwendet pro Konzentration zwei Pflanzen. Eine Auswertung der erzielten Abtötungsrate erfolgt 48 Stunden nach Applikation. Der Versuch wird bei 20 - 22°C und 60 % relativer Luftfeuchtigkeit durchgeführt.

Eine 80 - 100-%-ige Abtötung wird mit Verbindung Nr. 1 bei 100 ppm erzielt.

**Beispiel 9:**

Insektizide Kontaktwirkung: Aphis craccivora

In Töpfen angezogene Bohnenpflanzen (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 50 und 100 ppm der zu prüfenden Verbindung bis zur Tropfnässe besprüht. Man verwendet pro Test-Verbindung und pro Konzentration zwei Pflanzen, und eine Auswertung der erzielten Abtötungsrate erfolgt nach weiteren 24 Stunden.

Eine 80 - 100-%-ige Abtötung wird mit den Verbindungen Nr. 1, 2, 3 und 4 bei 50 ppm und mit Verbindung Nr. 5 bei 100 ppm erzielt.

**Beispiel 10:**

Insektizide Blattpenetrations-Wirkung: Aphis craccivora

In etwa 8 cm hohe Kunststoffbecher (Durchmesser etwa 6 cm) wird ein passender kleiner Zweig von Vicia faba (Saubohne) gelegt der mit Blattläusen der Spezies Aphis craccivora stark infiziert ist. Der Becher wird mit einem Kunststoffdeckel, der in der Mitte eine ausgestanzte Öffnung von 2 cm Durchmesser aufweist, bedeckt. Auf die in dem Deckel befindliche Öffnung wird ein Blatt einer Vicia faba-Pflanze gelegt, ohne dieses Blatt von der eingetopften Pflanze abzutrennen. Das Blatt wird dann mit einem zweiten Lochdeckel auf dem Becher über der Öffnung des ersten Deckels fixiert. Von der Unterseite her, d. h. durch die Öffnung des ersten Deckels hindurch, infizieren nun die in dem Becher befindlichen Blattläuse das obenliegende Blatt der Futterpflanze. Auf der Oberseite wird auf das Blatt eine wässrige Zubereitung des zu prüfenden Wirkstoffes in einer Konzentration von 100 ppm mittels eines Pinsels gleichmässig aufgetragen. Es wird geprüft, ob die einseitig auf die Oberseite des Blattes der Futterplanze aufgetragene Testsubstanz in genügender Menge durch das Blatt hindurch auf dessen Unterseite diffundiert, um die dort saugenden Blattläuse abzutöten.

Der Versuch wird bei etwa 20°C und 60 % relativer Luftfeuchtigkeit durchgeführt. Die Auswertung auf %-Mortalität erfolgt 48 Stunden nach Wirkstoffapplikation.

Die Verbindungen Nr. 1, 2 und 4 zeigen in diesem Test eine 80 - 100-%-ige Wirkung bei 100 ppm.

**Patentansprüche** für die Vertragsstaten: BE, CH, DE, FR, GB, IT, LI, NL, SE

1. Verwendung einer Verbindung der Formel I

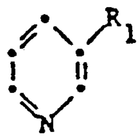 (I) ,

worin $R_1$ 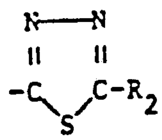 oder 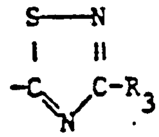 ,

und $R_2$ und $R_3$ Wasserstoff oder $C_1$-$C_4$-Alkyl bedeuten, oder eines diese Verbindung enthaltenden Mittels zur Bekämpfung von Pflanzen und Tiere befallenden Insekten und Vertretern der Ordnung Akarina.

2. Verwendung gemäss Anspruch 1 einer Verbindung der Formel I, worin $R_2$ und $R_3$ Wasserstoff oder $C_1$-$C_3$-Alkyl bedeuten.

3. Verwendung gemäss Anspruch 1 einer Verbindung der Formel

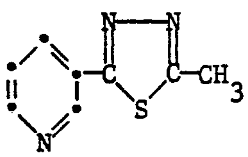 .

4. Verwendung gemäss Anspruch 1 einer Verbindung der Formel

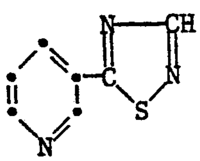 .

5. Verwendung gemäss Anspruch 1 einer Verbindung der Formel

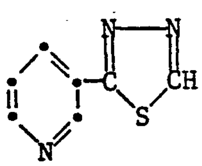 .

6. Verbindung der Formel I

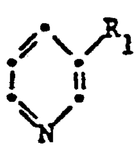 (I) ,

worin $R_1$ 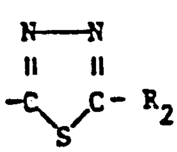 oder 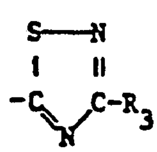

$R_2$ Wasserstoff oder $C_2$-$C_4$-Alkyl und

$R_3$ $C_1$-$C_4$-Alkyl bedeuten.

7. Verbindung der Formel I gemäss Anspruch 6, worin $R_2$ Wasserstoff oder $C_2$-$C_3$-Alkyl und $R_3$ $C_1$-$C_3$-Alkyl bedeuten.

8. Verbindung der Formel I gemäss Anspruch 7, worin $R_1$

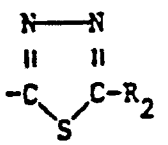 und

$R_2$ Wasserstoff oder $C_2$-$C_3$-Alkyl bedeuten.

9. Verbindung gemäss Anspruch 8 der Formel

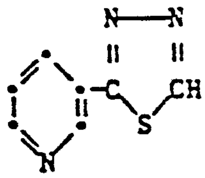

10. Verbindung gemäss Anspruch 8 der Formel

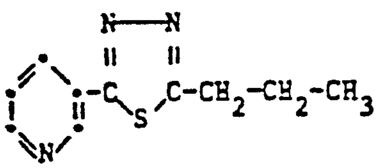

11. Verbindung gemäss Anspruch 7 der Formel

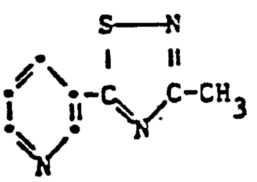

12. Verfahren zur Herstellung einer Verbindung der Formel I

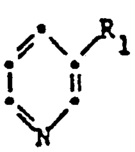 (I) ,

worin $R_1$ 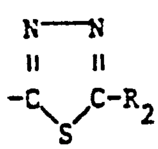

und $R_2$ Wasserstoff oder $C_2$-$C_4$-Alkyl bedeuten, dadurch gekennzeichnet, dass man eine Verbindung der Formel II

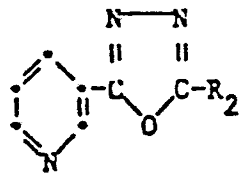

(II)

oder eine Verbindung der Formel III

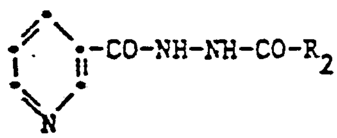

(III) ,

worin $R_2$ Wasserstoff oder $C_2$-$C_4$-Alkyl bedeutet, mit $P_2S_5$ umsetzt.

13. Verfahren zur Herstellung einer Verbindung der Formel I

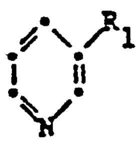

(I)

worin $R_1$

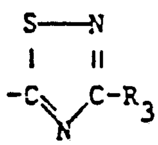

und $R_3$ $C_1$-$C_4$-Alkyl bedeuten, dadurch gekennzeichnet, dass man eine Verbindung der Formel IV

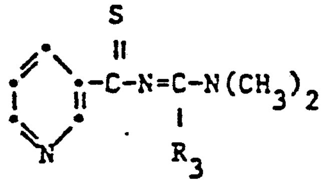

IV (III)

worin $R_3$ $C_1$-$C_4$-Alkyl bedeutet, in Gegenwart von Hydroxylamin-O-Sulfonsäure einem Ringschluss unterwirft.

**Patentansprüche** für den Vertragsstat: AT

1. Verwendung einer Verbindung der Formel I

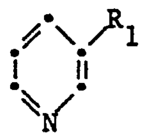

(I) ,

worin $R_1$ 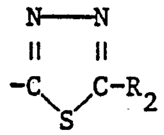 oder 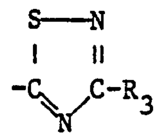 ,

und $R_2$ und $R_3$ Wasserstoff oder $C_1$-$C_4$-Alkyl bedeuten, oder eines diese Verbindung enthaltenden Mittels zur Bekämpfung von Pflanzen und Tiere befallenden Insekten und Vertretern der Ordnung Akarina.

2. Verwendung gemäss Anspruch 1 einer Verbindung der Formel I, worin $R_2$ und $R_3$ Wasserstoff oder $C_1$-$C_3$-Alkyl bedeuten.

3. Verwendung gemäss Anspruch 1 einer Verbindung der Formel

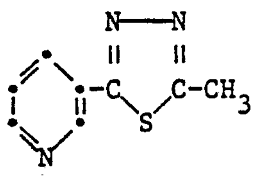

.

4. Verwendung gemäss Anspruch 1 einer Verbindung der Formel

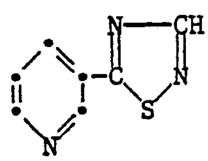

.

5. Verwendung gemäss Anspruch 1 einer Verbindung der Formel

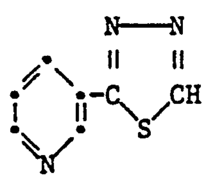

.

6. Verfahren zur Herstellung einer Verbindung der Formel I

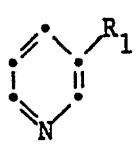

(I) ,

worin $R_1$

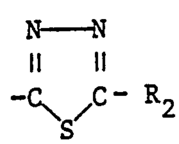

und $R_2$ Wasserstoff oder $C_2$-$C_4$-Alkyl bedeuten, dadurch gekennzeichnet, dass man eine Verbindung der Formel II

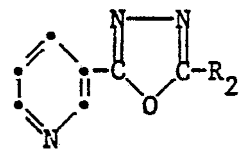

(II)

oder eine Verbindung der Formel III

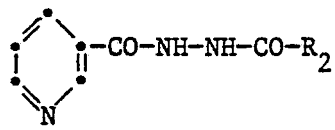

(III) ,

worin $R_2$ Wasserstoff oder $C_2$-$C_4$-Alkyl bedeutet, mit $P_2S_5$ umsetzt.

7. Verfahren nach Anspruch 6 zur Herstellung einer Verbindung der Formel I, worin $R_2$ Wasserstoff oder $C_2$-$C_3$-Alkyl bedeutet.

8. Verfahren zur Herstellung einer Verbindung der Formel I

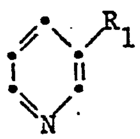

(I) ,

worin $R_1$

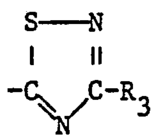

und $R_3$ $C_1$-$C_4$-Alkyl bedeuten dadurch gekennzeichnet, dass man eine Verbindung der Formel IV

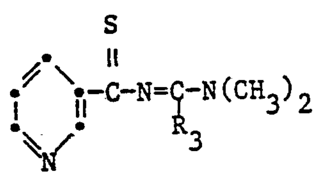

(IV) ,

worin $R_3$ $C_1$-$C_4$-Alkyl bedeutet, in Gegenwart von Hydroxylamin-O-Sulfonsäure einem Ringschluss unterwirft.

9. Verfahren nach Anspruch 8 zur Herstellung einer Verbindung der Formel I, worin $R_3$ $C_1$-$C_3$-Alkyl bedeutet.

10. Verfahren gemäss Anspruch 6 zur Herstellung der Verbindung der Formel

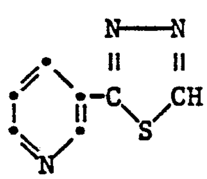

.

11. Verfahren gemäss Anspruch 7 zur Herstellung der Verbindung der Formel

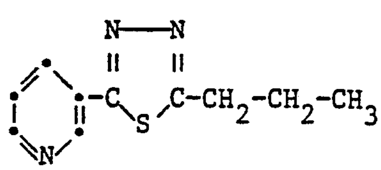

.

12. Verfahren gemäss Anspruch 9 zur Herstellung der Verbindung der Formel

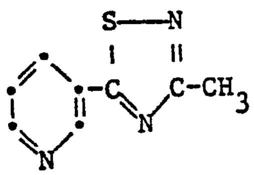

.

**Claims** for all contracting states except AT

1. The use of a compound of the formula I

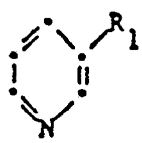

(I)

wherein $R_1$ is

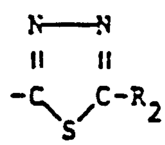

or

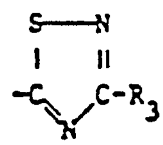

,

and $R_2$ and $R_3$ are hydrogen or $C_1$-$C_4$alkyl, or of a composition containing such a compound, for controlling representatives of the order Acarina and insects that attack plants and animals.

2. The use according to claim 1 of a compound of the formula I wherein $R_2$ and $R_3$ are hydrogen or $C_1$-$C_3$alkyl.

3. The use according to claim 1 of a compound of the formula

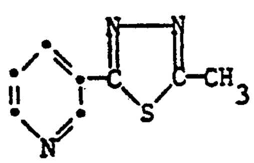

.

4. The use according to claim 1 of a compound of the formula

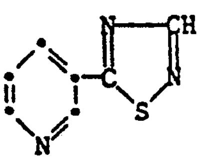

.

5. The use according to claim 1 of a compound of the formula

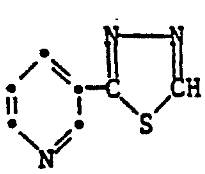

.

6. A compound of the formula I

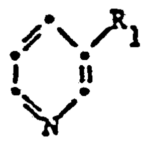

(I)

wherein $R_1$ is

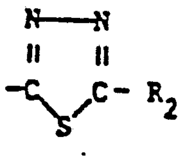

or

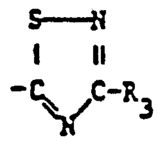

$R_2$ is hydrogen or $C_2$-$C_4$alkyl, and
$R_3$ is $C_1$-$C_4$alkyl.

7. A compound of the formula I according to claim 6 wherein $R_2$ is hydrogen or $C_2$-$C_3$alkyl and $R_3$ is $C_1$-$C_3$alkyl.

8. A compound of the formula I according to claim 7 wherein $R_1$ is

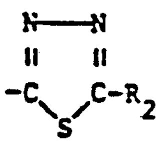

and
$R_2$ is hydrogen or $C_2$-$C_3$alkyl.

9. A compound according to claim 8 of the formula

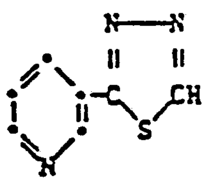

.

10. A compound according to claim 8 of the formula

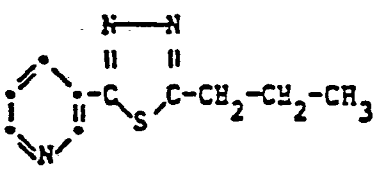

.

11. A compound according to claim 7 of the formula

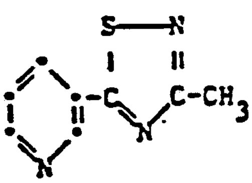

.

12. A process for the preparation of a compound of the formula I

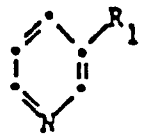

(I) ,

wherein $R_1$ is 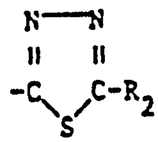

and $R_2$ is hydrogen or $C_2$-$C_4$alkyl, which comprises reacting a compound of the formula II

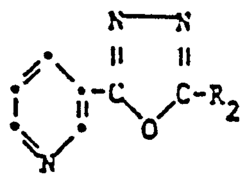

(II)

or a compound of the formula III

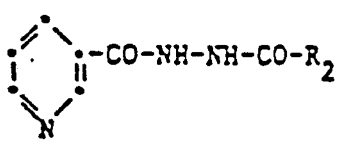

(III),

wherein $R_2$ is hydrogen or $C_2$-$C_4$alkyl, with $p_2S_5$.

13. A process for the preparation of a compound of the formula I

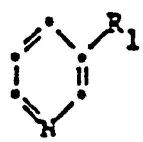

(I)

wherein $R_1$ is 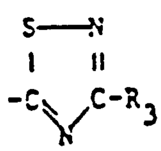

and $R_3$ is $C_1$-$C_4$alkyl, which comprises cyclising a compound of the formula III

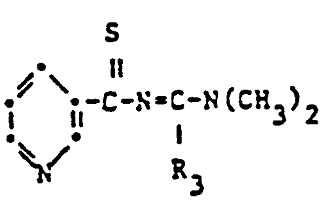

(III)

wherein $R_3$ is $C_1$-$C_4$alkyl, in the presence of hydroxylamine-O-sulfonic acid.

**Claims** for the contracting state: AT

1. The use of a compound of the formula I

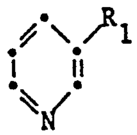

(I) ,

wherein $R_1$ is 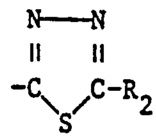 or 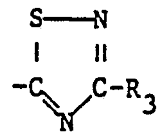 ,

and $R_2$ and $R_3$ are hydrogen or $C_1$-$C_4$alkyl, or of a composition containing such a compound, for controlling representatives of the order Acarina and insects that attack plants and animals.

2. The use according to claim 1 of a compound of the formula 1 wherein $R_2$ and $R_3$ are hydrogen or $C_1$-$C_3$alkyl.

3. The use according to claim 1 of a compound of the formula

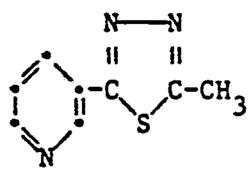 .

4. The use according to claim 1 of a compound of the formula

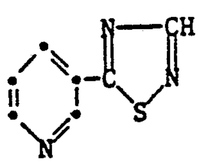 .

5. The use according to claim 1 of a compound of the formula

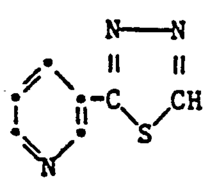 .

6. A process for the preparation of a compound of the formula I

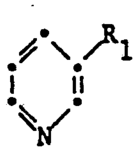

(I) ,

wherein $R_1$ is

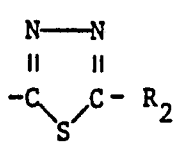

and $R_2$ is hydrogen or $C_2$-$C_4$alkyl, which comprises reacting a compound of the formula II

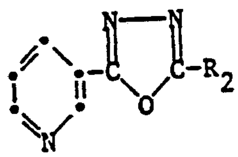 (II)

or a compound of the formula III

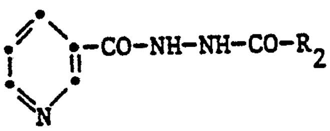 (III),

wherein $R_2$ is hydrogen or $C_2$-$C_4$alkyl, with $P_2S_5$.

7. A process according to claim 6 for the preparation of a compound of the formula I wherein $R_2$ is hydrogen or $C_2$-$C_3$alkyl.

8. A process for the preparation of a compound of the formula I

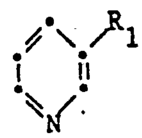 (I)

wherein $R_1$ is 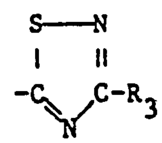

and $R_3$ is $C_1$-$C_4$alkyl, which comprises cyclising a compound of the formula IV

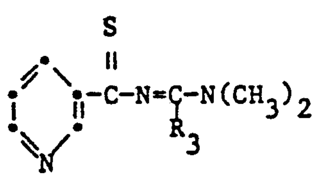 (IV)

wherein $R_3$ is $C_1$-$C_4$alkyl, in the presence of hydroxylamine-O-sulfonic acid.

9. A process according to claim 8 for the preparation of a compound of the formula I wherein $R_3$ is $C_1$-$C_3$alkyl.

10. A process according to claim 6 for the preparation of a compound of the formula

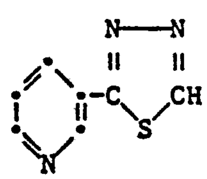 .

11. A process according to claim 7 for the preparation of a compound of the formula

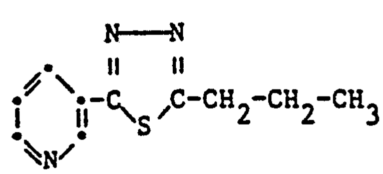 .

12. A process according to claim 9 for the preparation of a compound of the formula

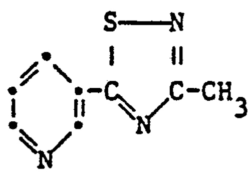

.

**Revendications** pour les états contractants: BE, CH, DE, FR, GB, IT, LI, NL, SE

1. Utilisation d'un composé de formule I

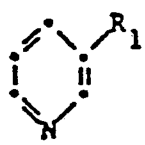

(I) ,

dans laquelle $R_1$ représente

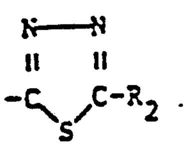

ou

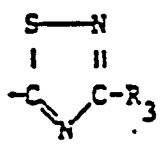

,

et $R_2$ représentent l'hydrogène ou un alkyle $C_1$-$C_4$, ou d'un moyen contenant l'un de ces composes pour combattre des insectes infestant des plantes ou des animaux, et des représentsnts de l'ordre acarina.

2. Utilisation selon la revendication 1 d'un composé de formule I, dans laquelle $R_2$ et $R_3$ représentent l'hydrogène ou un alkyle $C_1$-$C_3$.

3. Utilisation selon la revendication 1 d'un composé de formule

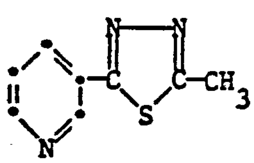

.

4. Utilisation selon la revendication 1 d'un composé de formule

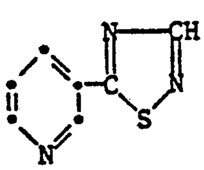

.

5. Utilisation selon la revendication 1 d'un composé de formule

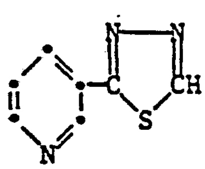

.

6. Composé de formule I

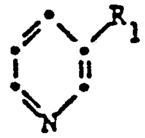

(I) ,

dans laquelle $R_1$ est

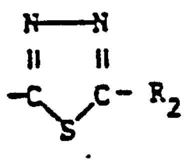

ou

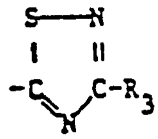

$R_2$ représente l'hydrogène ou un alkyle $C_2$-$C_4$ et
$R_3$ représente un alkyle $C_1$-$C_4$.

7. Composé de formule I selon la revendication 6, dans laquelle $R_2$ représente l'hydrogène ou un alkyle $C_2$-$C_3$ et $R_3$ un alkyle $C_1$-$C_3$.

8. Composé de formule I selon la revendication 7 dans laquelle $R_1$ représente

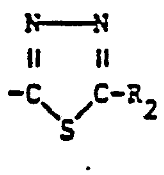

et $R_2$ l'hydrogène ou un alkyle $C_2$-$C_3$.

9. Composé selon la revendication 8 de formule

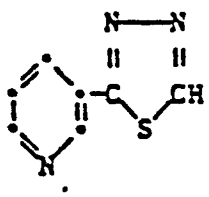

10. Composé selon la revendication 8 de formule

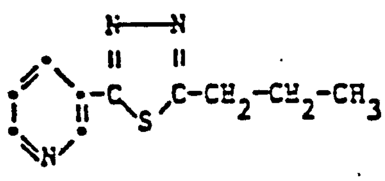

11. Composé selon la revendication 7 de formule

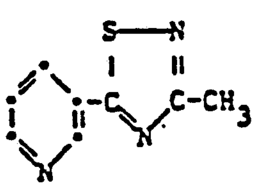

12. Procédé de préparation d'un composé de formule I

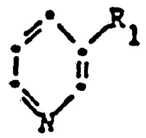

(I) ,

dans laquelle $R_1$ représente

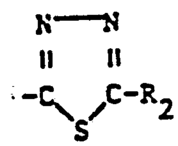

et $R_2$ représente l'hydrogène ou un alkyle $C_2$-$C_4$ caractérisé en ce qu'on fait réagir avec $P_2S_5$ un composé de formule II

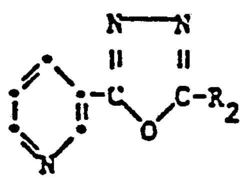

(II)

ou un composé de formule III

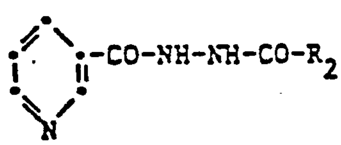

(III) ,

dans laquelle $R_2$ représente l'hydrogène ou un alkyle $C_2$-$C_4$.

13. Procédé de préparation d'un composé de formule I

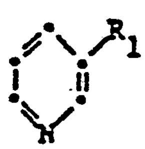

(I) .

dans laquelle $R_1$ représente

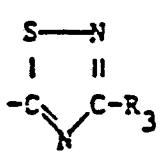

et $R_3$ représente un alkyle $C_1$-$C_4$, caractérisé en ce qu'on soumet un composé de formule III

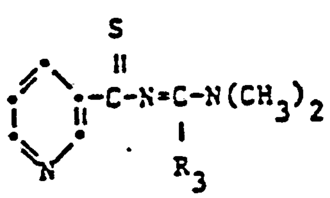

(III) .

dans laquelle $R_3$ représente un alkyle $C_1$-$C_4$, à une cyclisation en présence d'acide hydroxylamine-O-sulfonique.

**Revendications** pour l'état contractant: AT

1. Utilisation d'un composé de formule I

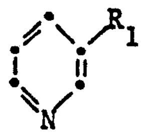

(I) ,

dans laquelle $R_1$ représente

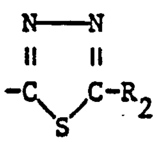

ou

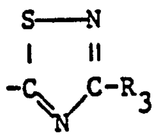

,

et $R_2$ et $R_3$ représentent l'hydrogène ou un alkyle $C_1$-$C_4$, ou d'un moyen contenant l'un de ces composés pour combattre des insectes infestant des plantes ou des animaux, et des représentants de l'ordre acarina.

2. Utilisation selon la revendication 1 d'un composé de formule I, dans laquelle $R_2$ et $R_3$ représentent l'hydrogène ou un alkyle $C_1$-$C_3$.

3. Utilisation selon la revendication 1 d'un composé de formule

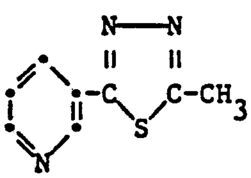

4. Utilisation selon la revendication 1 d'un composé de formule

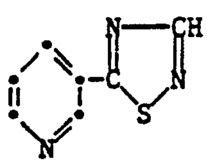

5. Utilisation selon la revendication 1 d'un composé de formule

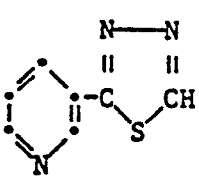

6. Procédé de préparation d'un composé de formule I

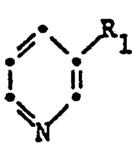

(I) ,

dans laquelle $R_1$ représente

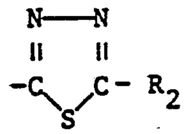

et $R_2$ représente l'hydrogène ou un alkyle $C_2$-$C_4$ caractérisé en ce qu'on fait réagir avec $P_2S_5$ un composé de formule II

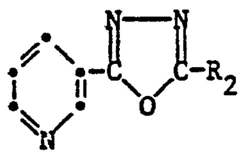

(II)

ou un composé de formule III

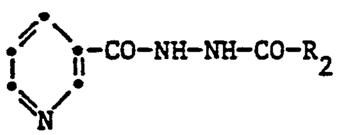

(III) ,

dans laquelle $R_2$ représente l'hydrogène ou un alkyle $C_2$-$C_4$.

7. Procédé selon la revendication 6 de préparation d'un composé de formule I dans laquelle $R_2$ représente l'hydrogène ou un alkyle $C_2$-$C_3$.

8. Procédé de préparation d'un composé de formule I

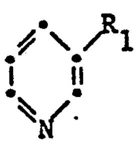

(I) ,

dans laquelle $R_1$ représente

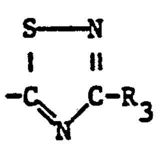

et $R_3$ représente un alkyle $C_1$-$C_4$, caractérisé en ce qu'on soumet un composé de formule IV

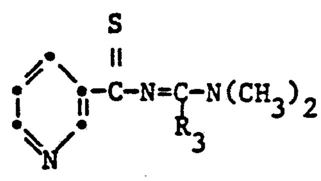

(IV) ,

dans laquelle $R_3$ représente un alkyle $C_1$-$C_4$, à une cyclisation en présence d'acide hydroxylamine-O-sulfonique.

9. Procédé selon la revendication 8 de préparation d'un composé de formule I, dans laquelle $R_3$ représente un alkyle $C_1$-$C_3$.

10. Procédé selon la revendication 6 de préparation du composé de formule

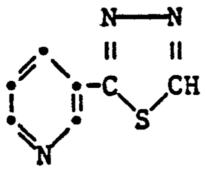

11. Procédé selon la revendication 7 de préparation du composé de formule

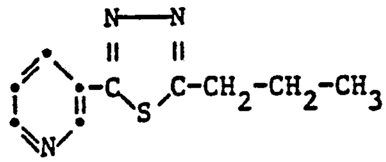

12. Procédé selon la revendication 9 de préparation du composé de formule